# EUROPEAN PATENT APPLICATION

(11) **EP 4 583 114 A1**
(43) Date of publication of application: **09.07.2025**
(21) Application number: 24150484.4
(22) Date of filing: 05.01.2024
(51) Int. Cl.: G16H 10/20, G16H 10/60, G16H 40/63, G16H 50/20, G16H 50/70

(54) **METHOD FOR FINDING SIMILAR PATIENTS, SYSTEM FOR FINDING SIMILAR PATIENTS AND COMPUTER PROGRAM PRODUCT**

(71) Applicant: Koninklijke Philips N.V., 5656 AG Eindhoven (NL)
(72) Inventor: VITORINO DE ALMEIDA, Vanda Lúcia, Eindhoven (NL); CZENKO, Marcin Ryszard, Eindhoven (NL); WINSEMIUS, Olivier, 5656AG Eindhoven (NL); JIA, Yiting, Eindhoven (NL); HENDRIKS, Cornelis Petrus, Eindhoven (NL)
(74) Representative: Philips Intellectual Property & Standards

(57) **Abstract**

The invention relates to a method (5) for finding similar patients. According to the method (5), clinical data with data sets of a plurality of patients is obtained (S 1). Further, a filtering selection is obtained (S2), wherein the filtering selection comprises at least one selected filter item (F1; F2; F3; F4-11; F4-t2) out of a plurality of filter items (F1 - F4; F4-t1 - F4-t3), wherein at least one out of the at least one selected filter item (F1; F2; F3; F4-t1; F4-t2) is a filter item (F4-t1; F4-t2; F4-t3) for a point in time (t1; t2; t3) of a time-series (t1 - 13), and for each of the at least one selected filter item (F1; F2; F3; F4-t1; F4-t2) a corresponding filter range (R1 - R3; R4-1; R4-2). Further, the data sets fulfilling the filtering selection are selected (S3) from the clinical data, a representation (6) of the data sets fulfilling the filtering selection is generated (S4) and the generated representation (6) is output (S5).

## Description

### FIELD OF THE INVENTION

The invention relates to patient similarity analyses in a clinical context, in particular to a method for finding similar patients. The invention further relates to a corresponding computer program product and to a system for finding similar patients.

### BACKGROUND OF THE INVENTION

Commonly, clinicians make decisions based on their clinical experience. Therefore, those who have not practiced for a while count with less background for making decisions. Furthermore, a complicating factor is the increasing amount of diagnostic data and treatment options, in combination with time pressure. Especially for complex patients - with multiple comorbidities - clinical guidelines often do not provide sufficient guidance.

This problem may be solved by providing medical decision support using similarity between patients, for example by selecting patients with similar characteristics who already went through the complete care path and show how these patients were treated and what were the outcomes, i.e., by a similarity analysis. This provides an explainable and interpretable decision support for clinicians. Further, using evidence from real-world persons may improve communication with patients (e.g., providing confidence, adherence, or to support shared decision making). Finally, the similarity analysis may help a hospital to monitor outcome improvement. A review of patient similarity for precision medicine is provided, e.g., in the article "Patient similarity for precision medicine: A systematic review" by E. Parimbelli, S. Marini, L. Sacchi, and R. Bellazzi (J. Biomed. Inform. 2018 Jul; 83:87-96).

### SUMMARY OF THE INVENTION

It is an object of the present invention to further improve the similarity analysis between patients. In particular, it is an object of the present invention to provide an improved method for finding similar patients, a system for finding similar patients and a corresponding computer program product.

The object of the present invention is solved by the subject matter of the independent claims, wherein further embodiments are incorporated in the dependent claims.

In an aspect of the present invention, a method for finding similar patients is provided. Said method may correspond to a patient similarity tool and may provide a similarity search. In particular, the method may provide a precise cohort selection to find similar patients.

According to the method, clinical data with data sets of a plurality of patients is obtained. In this context, the clinical data may be stored in a non-volatile memory and/or a persistent memory and may be obtained by reading the clinical data from said memory. Alternatively, or additionally, the clinical data may be imported from a data center, or may be provided via a wired or wireless connection.

The plurality of patients may comprise patients from a particular hospital, from many hospitals in a country, or even from many hospitals all over the world. Further, the data sets of a plurality of patients may comprise simulated patient parameters and/or simulated treatment outcomes, in order to increase the available number of data sets.

Further, a filtering selection is obtained. Said filtering selection may be a static filtering selection, but is preferably a dynamic filtering selection. The filtering selection comprises at least one selected filter item out of a plurality of filter items. Said filter items may comprise static patient characteristics, such as age, gender, weight, and/or height, biomarkers such as a histology type, inflammatory biomarkers (e.g., CRP, IL-#, CBC, MPO), cardiac biomarkers (e.g., hs-troponin, NT-proBNP), enzymatic biomarkers (e.g., ALT,AST, CK), hormonal biomarkers (e.g., TSH, Estrogen, Progesterone, Testosterone), and/or other metabolites and ions, and performed treatments, such as performed radiation treatments (e.g., mediastinal radiation) and/or given medication.

At least one out of the at least one selected filter item is a filter item for a point in time of a time-series. The time-series may also be referred to as history. In particular, the time-series may be a time-series of a biomarker. As an example, in oncological treatments, the time-series may be a time-series of a troponin biomarker. The time-series may be given as a function of actual time, e.g., hours, days or weeks, preferably with an appropriate binning. Also, the points in time may represent averages over a period of time. Alternatively, e.g., for the above example of oncological treatments, the time-series may be given in dependence on the oncological treatment cycles (e.g., baseline, C2, C3, ...). Including said time-series in the patient similarity analysis may help understanding whether a disease is improving or worsening, evaluating whether a therapy is adequate, and/or checking a patient's stability. Including the time-series may also improve decisions on further biomarker testing, in particular to avoid unnecessary testing, or timely decisions on additional medical imaging, e.g., in case of an increased risk, which may improve the patient's outcomes.

As an example, many of the existing biomarkers such as metabolites in body fluids usually have rather short half-lives, typically less than one or two days. Besides the point measurement, the time-series behavior of these biomarkers may be used to infer the clinical evolution or stability of the patient. Therefore, time-series of such biomarkers can help a clinician understanding whether the patient is stable, improving or worsening as response to a specific therapy. A visualization of the time -series can also help clinicians identifying the inflexion point in specific cases which is crucial for a fast intervention and optimization of outcomes.

As another example, two patients might have the same biomarker value, for example an average value from several oncological treatment cycles, or a value from a point measurement. However, one patient might have an increasing trend while the other patient might have a declining trend. So, the trend goes away or towards, respectively, a cut-off value which is used to take a disease management decision. Including the time-series will therefore help taking the disease management decision.

The filtering selection further comprises a corresponding filter range for each of the at least one selected filter item. With said filter range, a cohort selection is performed. The filter items may distinguish the data sets based on continuous values for the filter item or on categorical outcomes (i.e., discrete values) for the filter item.

The method further comprises selecting, from the clinical data, the data sets fulfilling the filtering selection. In this context, fulfilling the filtering selection means that for each selected filter item, the value given in the respective data set is within the corresponding filter range. That is, if the filter ranges are chosen such that the data for a current patient is within said filter ranges, the selected data sets correspond to patients that are similar to the current patient.

Further, the method comprises generating a representation of the data sets fulfilling the filtering selection and outputting the generated representation. In said representation, patient characteristics and biomarkers may be displayed, to allow a comparison of the current patient with a cohort of similar patients. Clinicians may then use this patient similarity analysis to support their clinical decisions. The generated representation may be output to a user interface, in particular to a graphical user interface, allowing both the displaying and interacting with the data, in particular with the time-series data, in a patient similarity analysis.

As an example, cardiotoxicity in oncological treatments (e.g., chemotherapy, radiation therapy, target therapies, immunotherapy, and/or surgery) is considered. Here, the patient similarity analysis including the time-series may help to decide on diagnostic tests and patient monitoring to avoid unplanned interruptions of the therapy plan.

As another example, an intensive care unit (ICU) is considered. Here, time-series data may be available from a variety of bedside devices (mobile chest X-ray, patient monitor, mechanical ventilator, and/or ultrasound). When the patient's condition deteriorates, for example by developing acute respiratory distress syndrome, a new computed tomography (CT) scan might be desirable, however performing a CT scan may be difficult with ICU patients. Also here, the patient similarity analysis comprising the time-series may help a pulmonologist to decide on whether or not a new CT scan shall be performed.

According to an embodiment, the filtering selection is obtained as follows. First, a temporary filtering selection is set to an initial filtering selection. In particular, said initial filtering selection may have no selected filter items. Alternatively, the initial filtering selection may have a predetermined selection of filter items, with filter ranges pre-selected around the data of the current patient. Then, the following steps are performed iteratively. This iterative procedure corresponds to a stepwise selection, which may lead to a precise cohort selection. The data sets fulfilling the temporary filtering selection are selected from the clinical data. For the initial filtering selection, this may be all data sets of the plurality of patients, i.e., the total patient population. As the iterative procedure advances, the temporary filtering selection will comprise more filter items and less data sets will be selected from the clinical data. A temporary representation of the data sets fulfilling the temporary filtering selection is then generated and output. Hence, a user may immediately see the results of the filtering selection. Then user input is obtained. Said user input may be obtained via a graphical user interface, using a touch screen and/or an input device, and/or using other input techniques, e.g., voice commands. Said user input may comprise a selection of a filter item. Said selection may be performed, e.g., by opening a list of possible filter items and performing a selection within this list, e.g., by placing checkmarks to the selected filter items. Further, the user input may comprise a selection of a filter range. In particular, said filter ranges may be selected for the selected filter items. The filter ranges may be selected, e.g., by moving end points of a slide bar, or by entering numerical values. It is also possible that a filter range is automatically chosen once a filter item has been selected, in particular such that the filter range contains the current patient's data. Said automatically selected filter range may then be adjusted manually later on. Further, the user input may comprise a selection of a time-series. Hence, the time-series may be chosen only when they are needed. When the time-series are not needed, they may stay hidden, in order to prevent a user from losing overview, to prevent the cohort selection from being too difficult due to too many items to choose from, and/or to improve the user's experience. Also, when the time-series are not needed, they may be skipped in the selection of the data sets, such that a corresponding computation speed goes up. Once the user input has been obtained, the temporary filtering selection is updated based on the obtained user input. This iterative procedure is repeated until the user has finished changing the temporary filtering selection. When the user has finished changing the temporary filtering selection, the filtering selection is set to the temporary filtering selection, and the step of obtaining a filtering selection is completed. With this method, an interactive way of performing the patient similarity analysis is provided, allowing a user to make a precise cohort selection.

According to an embodiment, the representation and/or the temporary representation is expanded upon the selection of a time-series. In case that, for example, filter items are given in columns, said expansion means the addition of extra columns for the time-series. This is particularly useful for short time-series, i.e., for time-series with a small number of points in time, such that the representation is not overwhelming yet all chosen filter items are within one representation.

Alternatively, a sub-representation is generated upon the selection of a time-series. Said sub-representation includes filter items for the time-series. As an example, the sub-representation may be presented in a new window of a graphical user interface. If, within the time-series, a time-series is selected, another sub-representation may be generated and display, e.g., in a third or a fourth window of the graphical user interface. By moving the time-series into the sub-representation, the temporary representation is not cluttered with the time-series data, hence more clearness is provided.

According to an embodiment, numerical values are replaced by categorical outcomes for the filter items for the points in time of the time-series. That is, continuous values are replaced by discrete values for these filter items. Such discrete values are based on (originally) continuous values and may be categorized as, e.g., "low, normal, elevated", "decreasing, stable, increasing", or "below, at, above a threshold or a cut-off value". This may simplify the overview for the user and improve the selection of the corresponding filter ranges.

According to an embodiment, the temporary representation and/or the sub-representation comprise indications of available time-series. In particular, said indications may correspond to specific filter items for which a time-series is available. As an example, the indication may be an icon on a graphical user interface and the time-series can be chosen by selecting said icon, e.g., by clicking on said icon. This way of selecting time-series is both easy and intuitive. Alternatively, or additionally, time-series may be chosen from a list of available time-series.

According to an embodiment, the representation, the temporary representation and/or the sub-representation comprise a Sankey diagram. Sankey diagrams are especially well suited for the presentation of a similarity analysis, and allow an intuitive pathway selection. Alternatively, or additionally, the representation, the temporary representation and/or the sub-representation comprise a clinical nomogram. This is also well suited for the presentation of a similarity analysis.

According to an embodiment, the representation, the temporary representation and/or the sub-representation comprise an outcome analysis. Said outcome analysis may comprise patient similarity results, i.e., the clinical outcomes for the patients whose data fulfills the respective filtering selection. Examples for said outcomes are cardiotoxicities, mortality rates, or interruptions of the therapy plan. With said outcomes, clinicians may directly interpret the results of the similarity analysis and treat a patient based on the analysis of said outcomes.

According to an embodiment, outputting the representation, the temporary representation and/or the sub-representation comprises outputting the representation, the temporary representation and/or the sub-representation, respectively, to an output interface. Said output interface may be an interface to a display, such as a graphics interface. Alternatively, or additionally, outputting the representation, the temporary representation and/or the sub-representation comprises displaying the representation, the temporary representation and/or the sub-representation, respectively, on a display. Said display may be the display of a graphical user interface and may be a stationary display or a display of a mobile device. In either case, the representation may be visually displayed, such that a clinician can easily and intuitively view the similarity analysis.

According to an embodiment, the method further comprises outputting the data sets fulfilling the filtering selection. Said data sets may be displayed on a display, saved on a memory or forwarded to another device, such that a clinician can inspect the data sets of the similar patients in detail, to support his/her treatment decision.

In another aspect of the present invention, a system for finding similar patients is provided. Said system comprises an input unit, a processing unit and an output unit. In particular, the system may be a specifically configured computer. The system enables finding similar patients, may provide a similarity search and may correspond to a patient similarity tool. In particular, the system may provide a precise cohort selection to find similar patients.

The input unit is configured to obtain clinical data with data sets of a plurality of patients. In this context, the clinical data may be stored in a non-volatile memory and/or a persistent memory and may be obtained by reading the clinical data from said memory. Alternatively, or additionally, the clinical data may be imported from a data center, or may be provided via a wired or wireless connection.

The plurality of patients may comprise patients from a particular hospital, from many hospitals in a country, or even from many hospitals all over the world. Further, the data sets of a plurality of patients may comprise simulated patient parameters and/or simulated treatment outcomes, in order to increase the available number of data sets.

The input unit is further configured to obtain a filtering selection. Said filtering selection may be a static filtering selection, but is preferably a dynamic filtering selection. The filtering selection comprises at least one selected filter item out of a plurality of filter items. Said filter items may comprise static patient characteristics, such as age, gender, weight, and/or height, biomarkers such as a histology type, inflammatory biomarkers (e.g., CRP, IL-#, CBC, MPO), cardiac biomarkers (e.g., hs-troponin, NT-proBNP), enzymatic biomarkers (e.g., ALT,AST, CK), hormonal biomarkers (e.g., TSH, Estrogen, Progesterone, Testosterone), and/or other metabolites and ions, and performed treatments, such as performed radiation treatments (e.g., mediastinal radiation) and/or given medication.

At least one out of the at least one selected filter item is a filter item for a point in time of a time-series. The time-series may also be referred to as history. In particular, the time-series may be a time-series of a biomarker. As an example, in oncological treatments, the time-series may be a time-series of a troponin biomarker. The time-series may be given as a function of actual time, e.g., hours, days or weeks, preferably with an appropriate binning. Also, the points in time may represent averages over a period of time. Alternatively, e.g., for the above example of oncological treatments, the time-series may be given in dependence on the oncological treatment cycles (e.g., baseline, C2, C3, ...). Including said time-series in the patient similarity analysis may help understanding whether a disease is improving or worsening, evaluating whether a therapy is adequate, and/or checking a patient's stability. Including the time-series may also improve decisions on further biomarker testing, in particular to avoid unnecessary testing, or timely decisions on additional medical imaging, e.g., in case of an increased risk, which may improve the patient's outcomes.

The filtering selection further comprises a corresponding filter range for each of the at least one selected filter item. With said filter range, a cohort selection is performed. The filter items may distinguish the data sets based on continuous values for the filter item or on categorical outcomes (i.e., discrete values) for the filter item.

The processing unit is configured to select, from the clinical data, the data sets fulfilling the filtering selection. In this context, fulfilling the filtering selection means that for each selected filter item, the value given in the respective data set is within the corresponding filter range. That is, if the filter ranges are chosen such that the data for a current patient is within said filter ranges, the selected data sets correspond to patients that are similar to the current patient.

The processing unit is further configured to generate a representation of the data sets fulfilling the filtering selection. In said representation, patient characteristics and biomarkers may be provided, to allow a comparison of the current patient with a cohort of similar patients.

The output unit is configured to output the generated representation The generated representation may be output to a user interface, in particular to a graphical user interface, allowing both the displaying and interacting with the data, in particular with the time-series data, in a patient similarity analysis. Clinicians may then use this patient similarity analysis to support their clinical decisions.

According to an embodiment, the processing unit is further configured to set a temporary filtering selection to an initial filtering selection. In particular, said initial filtering selection may have no selected filter items. Alternatively, the initial filtering selection may have a predetermined selection of filter items, will filter ranges pre-selected around the data of the current patient. Further, the processing unit is configured to select the data sets fulfilling the temporary filtering selection from the clinical data. For the initial filtering selection, this may be all data sets of the plurality of patients, i.e., the total patient population. The processing unit is further configured to generate a temporary representation of the data sets fulfilling the temporary filtering selection. The output unit is further configured to output the generated temporary representation, such that a user may immediately see the results of the filtering selection. The input unit is further configured to obtain user input. Said user input may be obtained via a graphical user interface, using a touch screen and/or an input device, and/or using other input techniques, e.g., voice commands. Said user input may comprise a selection of a filter item. Said selection may be performed, e.g., by opening a list of possible filter items and performing a selection within this list, e.g., by placing checkmarks to the selected filter items. Further, the user input may comprise a selection of a filter range. In particular, said filter ranges may be selected for the selected filter items. The filter ranges may be selected, e.g., by moving end points of a slide bar, or by entering numerical values. It is also possible that a filter range is automatically chosen once a filter item has been selected, in particular such that the filter range contains the current patient's data. Said automatically selected filter range may then be adjusted manually later on. Further, the user input may comprise a selection of a time-series. Hence, the time-series may be chosen only when they are needed. When the time-series are not needed, they may stay hidden, in order to prevent a user from losing overview, to prevent the cohort selection from being too difficult due to too many items to choose from, and/or to improve the user's experience. Also, when the time-series are not needed, they may be skipped in the selection of the data sets, such that a corresponding computation speed goes up. The processing unit is further configured to update the temporary filtering selection based on the obtained user input. Finally, the processing unit is configured to set the filtering selection to the temporary filtering selection. Hence, the system provides an interactive way of performing the patient similarity analysis, allowing a user to make a precise cohort selection. Further details, embodiments and advantages of the system correspond to the details, embodiments and advantages of the above-described method for finding similar patients.

In yet another aspect of the present invention, a computer program product is provided. The computer program product comprises instructions which, when the program is executed by a computer, cause the computer to carry out the above-described method for finding similar patients. Hence, details, embodiments and advantages of the computer program product correspond to the details, embodiments and advantages of the method for finding similar patients.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of a system for finding similar patients;
Fig. 2 shows a flowchart of an embodiment of a method for finding similar patients;
Fig. 3 shows a flowchart of an embodiment of the step of obtaining a filtering selection;
Fig. 4 shows an example for a representation of data sets;
Fig. 5 shows an example for a representation of data sets with an expanded time-series; and
Fig. 6 shows an example for a sub-representation of data sets.

In the Figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

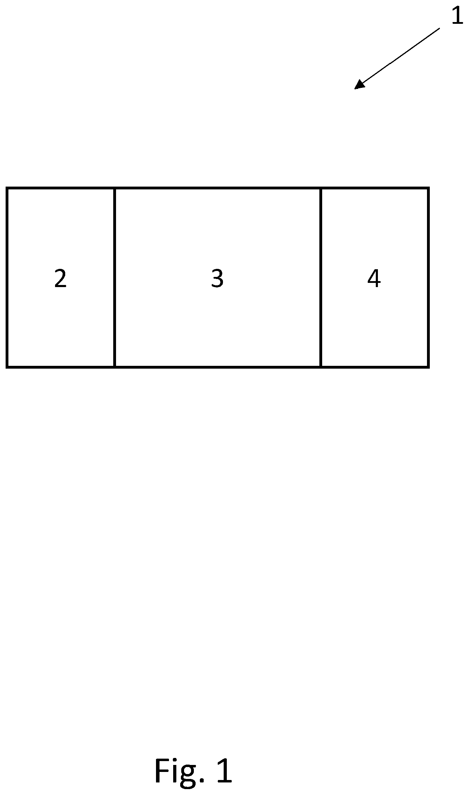

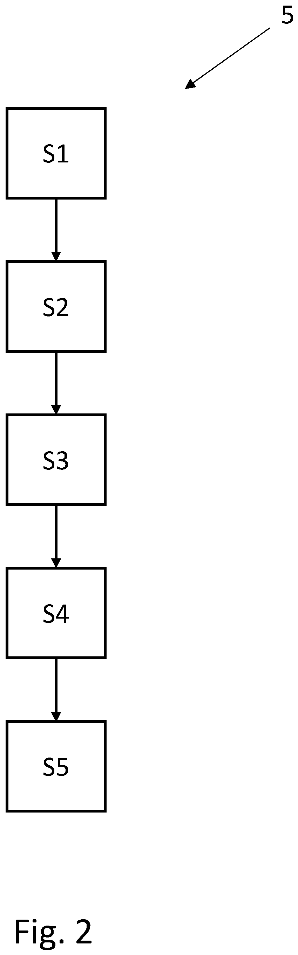

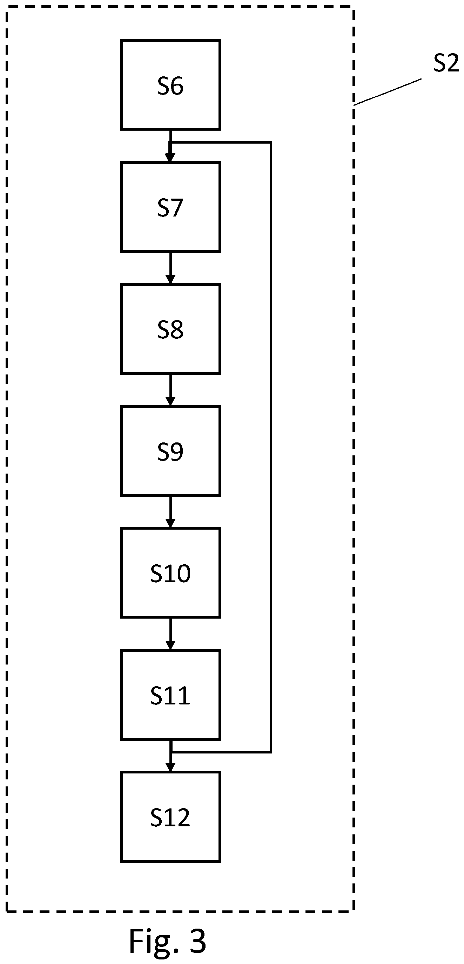

Fig. 1 shows a schematic view of a system 1 for finding similar patients. Said system 1 comprises an input unit 2, a processing unit 3, and an output unit 4. In particular, the system 1 may be based on a computer. The input unit 2, the processing unit 3 and the output unit 4 may be connected to one another using a wired and/or wireless connection. The system 1 for finding similar patients is configured to perform the method described in Figs 2 and/or 3.

Fig. 2 shows a flowchart of an embodiment of a method 5 for finding similar patients. Said method 5 may correspond to a patient similarity tool and may provide a similarity search. In particular, the method 5 may provide a precise cohort selection to find similar patients.

The method 5 comprises obtaining S 1 clinical data with data sets of a plurality of patients. In this context, the clinical data may be stored in a non-volatile memory and/or a persistent memory and may be obtained S1 by reading the clinical data from said memory. Alternatively, or additionally, the clinical data may be imported from a data center, or may be provided via a wired or wireless connection.

The plurality of patients may comprise patients from a particular hospital, from many hospitals in a country, or even from many hospitals all over the world. Further, the data sets of a plurality of patients may comprise simulated patient parameters and/or simulated treatment outcomes, in order to increase the available number of data sets.

The method 5 further comprises obtaining S2 a filtering selection. Said filtering selection may be a static filtering selection, but is preferably a dynamic filtering selection. The filtering selection comprises at least one selected filter item out of a plurality of filter items. Said filter items may comprise static patient characteristics, such as age, gender, weight, and/or height, biomarkers such as a histology type, inflammatory biomarkers (e.g., CRP, IL-#, CBC, MPO), cardiac biomarkers (e.g., hs-troponin, NT-proBNP), enzymatic biomarkers (e.g., ALT,AST, CK), hormonal biomarkers (e.g., TSH, Estrogen, Progesterone, Testosterone), and/or other metabolites and ions, and performed treatments, such as performed radiation treatments (e.g., mediastinal radiation) and/or given medication.

At least one out of the at least one selected filter item is a filter item for a point in time of a time-series. The time-series may also be referred to as history. In particular, the time-series may be a time-series of a biomarker. As an example, in oncological treatments, the time-series may be a time-series of a troponin biomarker. The time-series may be given as a function of actual time, e.g., hours, days or weeks, preferably with an appropriate binning. Also, the points in time may represent averages over a period of time. Alternatively, e.g., for the above example of oncological treatments, the time-series may be given in dependence on the oncological treatment cycles (e.g., baseline, C2, C3, ...). Including said time-series in the patient similarity analysis may help understanding whether a disease is improving or worsening, evaluating whether a therapy is adequate, and/or checking a patient's stability. Including the time-series may also improve decisions on further biomarker testing, in particular to avoid unnecessary testing, or timely decisions on additional medical imaging, e.g., in case of an increased risk, which may improve the patient's outcomes.

The filtering selection further comprises a corresponding filter range for each of the at least one selected filter item. With said filter range, a cohort selection is performed. The filter items may distinguish the data sets based on continuous values for the filter item or on categorical outcomes (i.e., discrete values) for the filter item.

The method further comprises selecting S3, from the clinical data, the data sets fulfilling the filtering selection. In this context, fulfilling the filtering selection means that for each selected filter item, the value given in the respective data set is within the corresponding filter range. That is, if the filter ranges are chosen such that the data for a current patient is within said filter ranges, the selected S3 data sets correspond to patients that are similar to the current patient.

Further, the method comprises generating S4 a representation of the data sets fulfilling the filtering selection and outputting S5 the generated representation. In said representation, patient characteristics and biomarkers may be displayed, to allow a comparison of the current patient with a cohort of similar patients. Clinicians may then use this patient similarity analysis to support their clinical decisions. The generated S4 representation may be output S5 to a user interface, in particular to a graphical user interface, allowing both the displaying and interacting with the data, in particular with the time-series data, in a patient similarity analysis.

Fig. 3 shows an embodiment of the step of obtaining S2 the filtering selection.

First, a temporary filtering selection is set S6 to an initial filtering selection. In particular, said initial filtering selection may have no selected filter items. Alternatively, the initial filtering selection may have a predetermined selection of filter items, will filter ranges pre-selected around the data of the current patient.

Then, the steps S7 to S11 are performed iteratively. This iterative procedure corresponds to a stepwise selection, which may lead to a precise cohort selection.

The data sets fulfilling the temporary filtering selection are selected S7 from the clinical data. For the initial filtering selection, this may be all data sets of the plurality of patients, i.e., the total patient population. As the iterative procedure advances, the temporary filtering selection will comprise more filter items and less data sets will be selected from the clinical data.

A temporary representation of the data sets fulfilling the temporary filtering selection is then generated S8 and output S9. Hence, a user may immediately see the results of the filtering selection.

Then user input is obtained S 10. Said user input may be obtained S 10 via a graphical user interface, using a touch screen and/or an input device, and/or using other input techniques, e.g., voice commands. Said user input may comprise a selection of a filter item. Said selection may be performed, e.g., by opening a list of possible filter items and performing a selection within this list, e.g., by placing checkmarks to the selected filter items. Further, the user input may comprise a selection of a filter range. In particular, said filter ranges may be selected for the selected filter items. The filter ranges may be selected, e.g., by moving end points of a slide bar, or by entering numerical values. It is also possible that a filter range is automatically chosen once a filter item has been selected, in particular such that the filter range contains the current patient's data. Said automatically selected filter range may then be adjusted manually later on. Further, the user input may comprise a selection of a time-series. Hence, the time-series may be chosen only when they are needed. When the time-series are not needed, they may stay hidden, in order to prevent a user from losing overview, to prevent the cohort selection from being too difficult due to too many items to choose from, and/or to improve the user's experience. Also, when the time-series are not needed, they may be skipped in the selection of the data sets, such that a corresponding computation speed goes up.

Once the user input has been obtained S 10, the temporary filtering selection is updated S11 based on the obtained S 10 user input.

This iterative procedure of steps S7 to S 11 is repeated until the user has finished changing the temporary filtering selection. When the user has finished changing the temporary filtering selection, the filtering selection is set S12 to the temporary filtering selection, and the step of obtaining S2 a filtering selection is completed. With this method, an interactive way of performing the patient similarity analysis is provided, allowing a user to make a precise cohort selection.

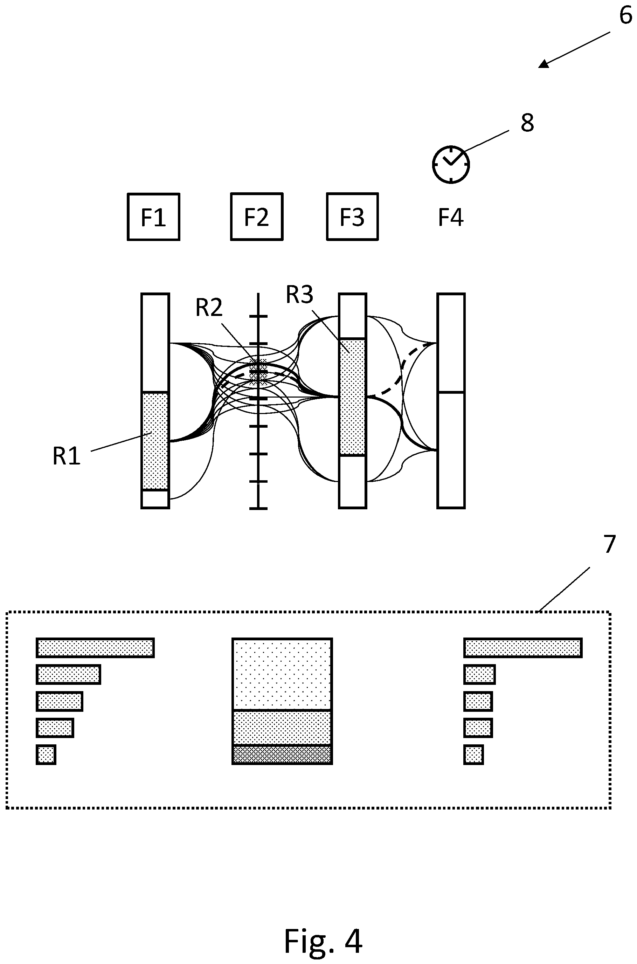

Fig. 4 shows an example of a representation 6 of data sets. Said representation is shown as a Sankey diagram, however, also a representation as a clinical nomogram may be used. The representation 6 may also be a temporary representation during the filtering selection as described above.

The representation 6 shows a plurality of filter items, here four filters items F1 to F4. It is to be understood that other numbers of filter items, in particular greater than four, are also possible. Out of these filter items, three filter items F1 to F3 have been selected, as indicated by the box around the filter item labels. Of course, other indications may also be possible. For filter items F1, F3 and F4, categorical outcomes are given, as indicated by the bars, whereas for filter item F2 numerical values are given. Further, for the selected filter items F1 - F3, filter ranges R1 - R3 are indicated by the shaded areas. Also, connecting lines are indicated, wherein the dashed line represents the data for a current patient, the thick solid lines corresponds to similar patients, i.e., patients with data sets fulfilling the filtering selection, and the thin lines correspond to other patients out of the plurality of patients whose data sets do not fulfil the filtering selection. Further, the representation 6 comprises an outcome analysis 7, where the clinical outcomes (e.g., cardiotoxicities, mortality rates, interruptions of therapy plan) for the selected data sets are shown.

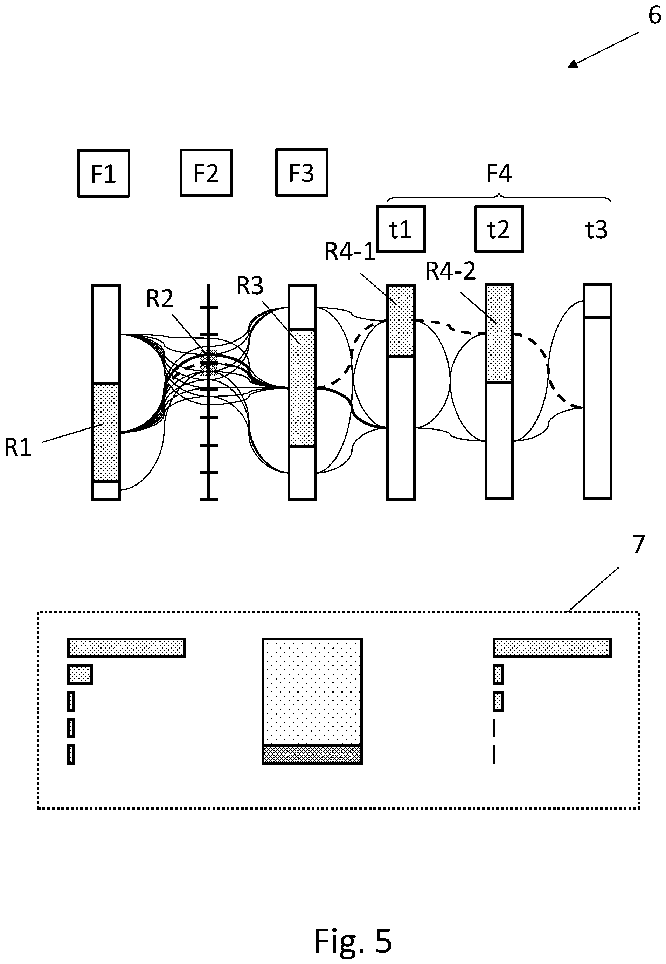

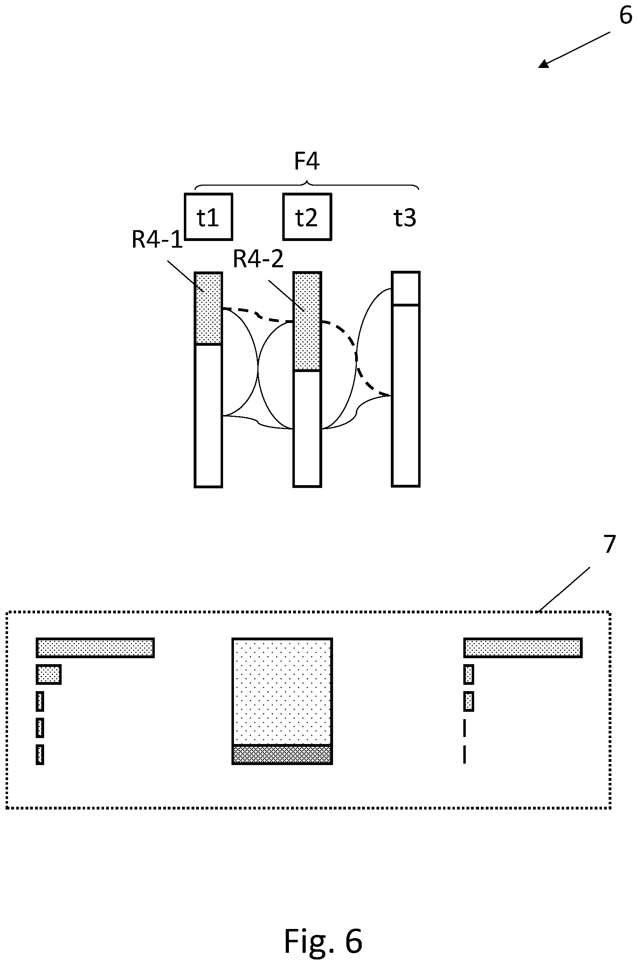

Moreover, the representation comprises an indication 8 of available time-series, here shown as a clock symbol. Upon selection of the time-series, e.g., by clicking on the indication, the representation 6 may be expanded, as shown in Fig. 5, or a sub-representation may be generated, as shown in Fig. 6.

As shown in Fig. 5, the representation 6 has been expanded by showing the points in time t1, t2, t3 for the time-series t1 - t3. Hence, there are now three filter items F4-t1, F4-t2 and F4-t3 for the points of time t1, t2 and t3, respectively. Out of these three filter items F4-t1, F4-t2 and F4-t3, two filter items F4-t1 and F4-t2 have been selected, and the ranges have been chosen as R4-1 and R4-2, respectively. Hence, a very precise cohort selection has been enabled.

Alternatively, as shown in Fig. 6, a sub-representation 9 that includes only the filter items F4-t1, F4-t2 and F4-t3 for the time-series t1 - t3 is generated. Especially for representations with many filter items, F1 - F3, F4-t1 - F4-t3, this provides a clearer representation.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 1: system for finding similar patients
- 2: input unit
- 3: processing unit
- 4: output unit
- 5: method for finding similar patients
- 6: representation
- 7: outcome analysis
- 8: indication of available time-series
- 9: sub-representation

- S1: obtaining clinical data
- S2: obtaining a filtering selection
- S3: selecting data sets
- S4: generating a representation
- S5: outputting the representation
- S6: setting a temporary filtering selection
- S7: selecting data sets
- S8: generating a temporary representation
- S9: outputting the temporary representation
- S10: obtaining user input
- S11: updating the temporary filtering selection
- S12: setting the filtering selection

- F1 - F4: filter items
- F4-t1 - F4-t3: filter items for points in time of a time-series
- t1 - t3: points in time of a time-series
- R1 - R3: filter ranges
- R4-1, R4-2: filter ranges

## Claims

1. A method (5) for finding similar patients, comprising:
obtaining (S 1) clinical data with data sets of a plurality of patients;
obtaining (S2) a filtering selection, wherein the filtering selection comprises:
at least one selected filter item (F1; F2; F3; F4-t1; F4-t2) out of a plurality of filter items (F1 - F4; F4-t1 - F4-t3), wherein at least one out of the at least one selected filter item (F1; F2; F3; F4-t1; F4-t2) is a filter item (F4-t1; F4-t2; F4-t3) for a point in time (t1; t2; t3) of a time-series (t1 - t3); and
for each of the at least one selected filter item (F1; F2; F3; F4-t1; F4-t2) a corresponding filter range (R1 - R3; R4-1; R4-2);
selecting (S3), from the clinical data, the data sets fulfilling the filtering selection;
generating (S4) a representation (6) of the data sets fulfilling the filtering selection; and
outputting (S5) the generated representation (6).

2. The method (5) according to claim 1, wherein the step of obtaining (S2) a filtering selection comprises:
setting (S6) a temporary filtering selection to an initial filtering selection;
performing the following steps iteratively:
selecting (S7), from the clinical data, the data sets fulfilling the temporary filtering selection;
generating (S8) a temporary representation (6) of the data sets fulfilling the temporary filtering selection;
outputting (S9) the generated temporary representation (6);
obtaining (S 10) user input, wherein the user input comprises at least one out of:
selection of a filter item (F1 - F4; F4-t1 - F4-t3);
selection of a filter range (R1 - R3; R4-1; R4-2); and
selection of a time-series (t1 - t3); and
updating (S 1 1) the temporary filtering selection based on the obtained user input; and
setting (S12) the filtering selection to the temporary filtering selection.

3. The method (5) according to claim 2, wherein, upon the selection of a time-series (t1 - t3), the representation (6) and/or the temporary representation (6) is expanded.

4. The method (5) according to claim 2, wherein, upon the selection of a time-series (t1 - t3), a sub-representation (9) is generated, wherein the sub-representation (9) includes filter items (F4-t1; F4-t2; F4-t3) for the time-series (t1 - t3).

5. The method (5) according to any one of claims 1 to 4, wherein, for the filter items (F4-t1; F4-t2; F4-t3) for the points in time (t1; t2; t3) of the time-series (t1 - t3), numerical values are replaced by categorical outcomes.

6. The method (5) according to any one of claims 2 to 5, wherein the temporary representation (6) and/or the sub-representation (9) comprise indications (8) of available time-series (t1 - t3).

7. The method (5) according to any one of claims 1 to 6, wherein the representation (6), the temporary representation (6) and/or the sub-representation (9) comprise a Sankey diagram and/or a clinical nomogram.

8. The method (5) according to any one of claims 1 to 7, wherein the representation (6), the temporary representation (6) and/or the sub-representation (9) comprise an outcome analysis (7).

9. The method (5) according to any one of claims 1 to 8, wherein outputting (S5; S9) the representation (6), the temporary representation (6) and/or the sub-representation (9) comprises outputting the representation (6), the temporary representation (6) and/or the sub-representation (9), respectively, to an output interface and/or displaying the representation (6), the temporary representation (6) and/or the sub-representation (9), respectively, on a display.

10. The method (5) according to any one of claims 1 to 9, further comprising:
outputting the data sets fulfilling the filtering selection.

11. A system (1) for finding similar patients, comprising:
an input unit (2), configured to:
obtain clinical data with data sets of a plurality of patients; and
obtain a filtering selection, wherein the filtering selection comprises:
at least one selected filter item (F1; F2; F3; F4-t1; F4-t2) out of a plurality of filter items (F1 - F4; F4-t1 - F4-t3), wherein at least one out of the at least one selected filter item (F1; F2; F3; F4-t1; F4-t2) is a filter item (F4-t1; F4-t2; F4-t3) for a point in time (t1; t2; t3) of a time-series (t1 - t3); and
for each of the at least one selected filter item (F1; F2; F3; F4-t1; F4-t2) a corresponding filter range (R1 - R3; R4-1; R4-2);
a processing unit (3), configured to:
select, from the clinical data, the data sets fulfilling the filtering selection; and
generate a representation (6) of the data sets fulfilling the filtering selection; and an output unit (4), configured to:
output the generated representation (6).

12. The system (1) according to claim 11, wherein
the processing unit (3) is further configured to:
set a temporary filtering selection to an initial filtering selection ;
select, from the clinical data, the data sets fulfilling the temporary filtering selection; and
generate a temporary representation (6) of the data sets fulfilling the temporary filtering selection;
the output unit (4) is further configured to:
output the generated temporary representation (6);
the input unit (2) is further configured to:
obtain user input, wherein the user input comprises at least one out of:
selection of a filter item (F1 - F4; F4-t1 - F4-t3);
selection of a filter range (R1 - R3; R4-1; R4-2); and
selection of a time-series (t1 - t3); and
the processing unit (3) is further configured to:
update the temporary filtering selection based on the obtained user input; and
set the filtering selection to the temporary filtering selection.

13. A computer program product comprising instructions which, when the program is executed by a computer, cause the computer to carry out the method (5) according to any one of claims 1 to 10.
